# EUROPÄISCHE PATENTSCHRIFT

(11) **EP 2 049 192 B1**
(45) Veröffentlichungstag und Bekanntmachung des Hinweises auf die Patenterteilung: **03.12.2014**
(21) Anmeldenummer: 07785657.3
(22) Anmeldetag: 20.07.2007
(51) Int. Cl.: A61M 39/10, A61F 2/06

(54) **VASKULÄRES RÖHRENFÖRMIGES TRANSPLANTAT**
TUBULAR VASCULAR TRANSPLANT
GREFFON VASCULAIRE TUBULAIRE

(30) Priorität: 02.08.2006 DE 102006036073
(43) Veröffentlichungstag der Anmeldung: 22.04.2009
(73) Patentinhaber: Heise, Michael, 07743 Jena (DE)
(72) Erfinder: HEIDENHAIN, Christoph, 10119 Berlin (DE)
(74) Vertreter: Czybulka, Uwe
(86) Internationale Anmeldenummer: PCT/DE2007/001289
(87) Internationale Veröffentlichungsnummer: WO 2008/014752

(56) Entgegenhaltungen:
- WO-A-97/43983
- US-A- 3 818 511
- US-A- 4 503 568

## Beschreibung

Die Erfindung bezieht sich auf ein vaskuläres röhrenförmiges Transplantat gemäß dem Oberbegriff des Patentanspruches 1.

Derartige Transplantate dienen allgemein zum Verbinden von Blut durchströmten. Gefäßen im menschlichen Körper. Das Transplantat wird an seinem ersten proximalen Ende mit einem Gefäß und mit seinem zweiten distalen Ende an einem anderen Ort desselben Gefäßes oder mit einem anderen Gefäß verbunden.

Ein Beispiel sind so genannte periphere Transplantate, die etwa dazu dienen, ein nicht mehr von Blut durchströmtes Gefäß mit Hilfe des Transplantates zu umgehen; auch Anwendungen im Rahmen einer End-zu-Seit-Anastomose sind möglich.

Ein weiteres Bespiel für ein solches vaskuläres röhrenförmiges Transplantat ist ein arteriovenösen, Shunt, das heißt eine Nebenschluss- oder Kurzschlussverbindung zwischen einer Arterie und einer Vene, meistens im Ober- oder Unterarm, um bei einer Dialyse einen operativen Gefäßzugang zu ermöglichen. Ein solcher Shunt wird vorgesehen, wenn die körpereigenen Venen eines Patienten nicht so gut ausgebildet sind, dass sie eine effektive Dialyse zulassen. Über einen arteriovenösen Shunt sind Durchflussraten bis zu 1000 Milliliter pro Minute (1000 ml/min) möglich. Bei einer Dialyse wird in das Transplantat in dem proximalen, das heißt der Arterie zugewandten Bereich eine erste Punktionsnadel eingeführt, von der das durch das Transplantat fließende Blut über ein Dialysegerät geleitet und von dort über eine zweite Punktionsnadel in dem dem distalen Ende zugewandten Bereich in das Transplantat zurückgeführt wird.

Vaskuläre röhrenförmige Transplantate werden meistens aus expandiertem Polytetrafluorethylen hergestellt und als ePTFE-Transplantat bezeichnet.

Ein generelles Problem solcher vaskulärer röhrenförmiger Transplantate ist die Ausbildung einer Stenose im distalen Bereich des Transplantats. Eine solche Stenose entwickelt sich meistens innerhalb der ersten sechs Monate bis zwei Jahre nach dem Einsetzen des Transplantats. Bei einem arteriovenösen Shunt entwickelt sich die Stenose an zwei Stellen der distalen Anastomose: Am Boden der empfangenden Vene entsteht in der Nähe der Anastomose eine Intimahyperpläsie, im Bereich des distalen Endes innerhalb des Transplantats bildet sich eine Pseudointima.

Ähnliche Probleme treten auch bei peripheren Transplantaten oder End-zu-Seit-Anastomosen auf.

Diese chronischen Veränderungen sind als unabhängig voneinander entstehende Phänomene zu betrachten. Die Ursache für beide Phänomene sind nach derzeitigem Stand er Wissenschaft unterschiedlich:
1. Die Intimahyperplasie, zum Beispiel an dem Boden der Vene bei einem arteriovenösen Shunt, entsteht durch sehr hohe Wandscherstresse im Bereich der Wand der Vene, die im Wesentlichen durch die mit hoher Geschwindigkeit schräg einfallende Zentralströmung des Bluts hervorgerufen werden.
2. Die Pseudointimahyperplasie im Bereich des Transplantates entsteht durch Totwasserzonen, so genannte Separationszonen, in denen das Blut nur sehr langsam oder gar nicht fließt. Über die Bildung von wandständigen Thrombosen kommt es zur Umwandlung der Thromben in eine Pseudointima, die letztlich zur Lumen-Einengung des Transplantates führt. Durch ausgedehnte Totwasser- und Wirbelbildungszonen im Bereich der aufgeweiteten Anastomose sind hier die Stenose besonderes ausgeprägt.

Das Problem von Stenosen, insbesondere venösen Stenosen, ist seit längerem bekannt.. Für einen arteriovenösen Shunt wurde das so genännte Venaflo®-Transplantat aus ePTFE entwickelt; vergl. auch die EP 1 011 521 B1, hier insbesondere die Figuren, 6A und 6B mit zugehöriger Beschreibung. Diese europäische Patentschrift wurde auch als DE 696 34 278 T2 veröffentlicht. Dieses bekannte Transplantat ist im distalen Bereich durch einen Flansch aufgeweitet, wodurch es hier zu einer Wirbelbildung kommt. Durch die Wirbel sollen die Scherstresse auf den Boden des empfangenden Gefäßes, z. B. einer Vene, abgeschwächt werden und damit die Ausbildung einer Intimahyperplasie verhindert werden. In verschiedenen Studien konnte gezeigt werden, dass die Stenoserate bei diesem bekannten Implantat gegenüber einfachen geraden End-zu-Seit-Transplantaten tatsächlich geringer ist. Andererseits kann sich jedoch bei diesem bekannten Transplantat im distalen Bereich eine ausgeprägte Pseudointima ausbilden. Dies könnte daran liegen, dass in der Aufweitung des Transplantats besonders große Wirbel- und Separationszonen entstehen.

Albert G. Hakain et al. veröffentlichten in der Zeitschrift Journal of Vascular Surgery, Band 37, Nr. 5, Mai 2003, Seiten 1032 ff einen Vorschlag für die Ausbildung eines arteriovaskulären Shunts im Bereich der venenseitigen Anastomose. Die gerade ePTFE-Prothese, wurde durch spezielle Schnitte in der Vene mit dieser so verbunden, dass sich im Venenbereich ein diffusorartiger Strömungsweg äusbildet. Wenn zudem der Eintrittswinkel zwischen der Prothese und der Vene nur wenige Grad, insbesondere etwa 15 Grad ist, können nach dem dortigen Bericht die Durchflussraten des Blutes, erhöht werden. Ebenso werden dadurch Turbulenzen vermieden. Ebenso zeigte sich auch nach 24 Monaten nur eine geringe Stenoseausbildung.

Im Folgenden soll die angesprochene Problematik für einen arteriovenösen Shunt diskutiert werden, wobei diese Probleme in ähnlicher Form auch bei peripheren vaskulären Transplantaten auftreten.

Um die Stenoserate zu verringern und die klinische Wirkung zu verbessern, müssten zwei Punkte verbessert werden:
1. Die extrem hohen Wandscherstresse, die durch den schräg einfallenden Zentralstrom des Blutes im Bereich der Venenwand hervorgerufen werden, müssten deutlich abgesenkt werden;
2. Die Ausbildung von Separationszonen innerhalb des Transplantats müsste verhindert werden, insbesondere im Bereich der venösen Anastomose.

Der Erfindung liegt daher die Aufgabe zugrunde, ein vaskuläres röhrenförmiges Transplantat der in Rede stehenden Art anzugeben, bei dem die Strömungsverhältnisse insbesondere im distalen Bereich des Transplantates so kontrolliert werden können, dass die Ausbildung von Stenosen und damit Ausbildungen einer Intimahyperplasie und einer Pseudointima möglichst verhindert bzw. deutlich vermindert werden.

Diese Aufgabe ist gemäß der Erfindung durch die Merkmale des Patentanspruches.1 gelöst.

Demgemäß erweitert sich das Lumen des Transplantates im Bereich seines distalen Endes stetig konisch und bildet dort einen Diffusor.

Durch den Einbau eines. Diffusors in dem distalen Bereich des Transplantats, bei einem arteriovenösen Shunt im Bereich des venösen Teiles dieses Shunts, kann die Strömungsgeschwindigkeit des Blutes kontrolliert gedrosselt und abgesenkt werden. Durch diese Strömungsverlangsamung werden deutlich geringere Scherstresse auf die Wand des empfangenden Gefäßes, zum Beispiel einer Vene, erzielt werden. Der Konuswinkel der Wand des Diffusors in Bezug auf die zentrale Strömungsrichtung des Blutes in dem Transplantat wird hierbei so gewählt, dass keine oder nur geringe Separationszonen auftreten, in denen das Blut nur sehr langsam fließt. Der Konuswinkel sollte kleiner als 10°, vorzugsweise im Bereich zwischen 6° und 7° gewählt werden, da es sonst wieder zur Flussseparation kommt und damit zur Entwicklung einer Pseudointima.

Mit dem Einbau eines Diffusors in dem distalen Bereich des Transplantates kann auf diese Weise sowohl die Entstehung einer Intimahyperplasie am Boden des empfangenden Gefäßes als auch einer Pseudointima im distalen Bereich des Transplantats verhindert beziehungsweise deutlich vermindert werden.

Eine weitere Steigerung dieses Effekts wird gemäß der Erfindung erreicht, indem in dem Diffusorbereich des Transplantates zumindest eine in Richtung der Zentralströmung des Blutes oder parallel hierzu gelegene Trennwand vorgesehen ist, die zwischen gegenüber liegenden Wandbereichen des Diffusors angeordnet ist.

Durch zumindest eine solche längs verlaufende Trennwand in dem Diffusorbereich des Transplantates wird die Blutströmung in zwei Hälften aufgeteilt, wobei jedoch in der Summe der Diffusoreffekt beibehalten wird. Die Trennwand ist bevorzugt eben.

Aus der US-PS 6,589,278 ist eine röhrenförmige vaskuläre Prothese beschrieben, die an ihrem distalen Ende eine Aufweitung zeigt, um die lokale Strömung des Blutes in diesem aufgeweiteten Bereich zu beeinflussen und zu verbessern. In diesem Bereich soll eine nicht laminare, teilweise turbulente Strömung erzeugt werden, um einen Scherstress im Bereich der Arterienwand aufzubauen. Hiermit soll auch eine Intimahyperplasie in der Arterie vermieden werden.

Der aufgeweitete Bereich dieser bekannten Prothese ist jedoch kein Diffusor, sondern weist spezielle konkave Wandgestaltungen aus, um die erwähnen speziellen Strömungszustände zu erreichen.

Gemäß der Erfindung wird durch die Ausbildung der Aufweitung im distalen Bereich der Prothese die Strömung in Richtung des weiten Ausganges in der Geschwindigkeit vermindert, wobei gleichzeitig der Druck ansteigt. Es werden keine turbulenten oder nicht-laminaren Strömungen erzeugt. Hierdurch können Ablagerungen in den Adern zuverlässig verhindert werden.

Es ist im Übrigen durchaus möglich, nicht nur eine Trennwand in dem Diffusorbereich, sondern mehrere gegebenenfalls gegen einander geneigte Trennwände einzusetzen, dieses insbesondere bei extremen Diffusorformen.

Es ist auch möglich, die Trennwand nicht durchgehend auszugestalten; eine oder zwei Trennwände können sich hierbei, ausgehend von. zumindest einem Wandbereich des Transplantates, segelartig in den Diffusor erstrecken. Werden zwei gegenüber liegende Trennwände vorgesehen, so kann zwischen deren inneren Rändern ein Zwischenraum verbleiben.

Beim Einsatz einer einzigen Trennwand in dem Diffusorbereich des Transplantates mit einem konischen Öffnungswinkel des Diffusors von etwa 7°, zeigte es sich völlig'überraschend, dass 25% bis 30% höhere Durchflussmengen erreicht wurden. Diese Ergebnisse konnten durch Untersuchungen an Silikonmodellen eines solchen Transplantates bestätigt werden. Die Silikonmodelle wurden hierbei in einem Modellkreislauf untersucht, der durch ein Kunstherz angetrieben wurde. Hierbei wurden auch gleichzeitig Laser gestützte Messungen der Strömungsgeschwindigkeiten durchgeführt, mit der die Berechnung der Scherstress-Werte möglich ist. Die Erklärung für diese Ergebnisse ist vermutlich, dass es durch die ebene Trennwand (oder gegebenenfalls durch mehrere Trennwände) zu einer Strömungsaufteilung und gleichzeitig zu einer Laminarisierung der Strömung in dem Diffusorbereich kommt, ohne dass hierbei Separationszonen auftreten, die zu hohen Energieverlusten führen. Diese sind gerade bei pulsartigen Strömungen und hochviskosen Flüssigkeiten, wie bei einem schlagenden Herzen und menschlichem Blut, besonders ungünstig, da sie bei jedem Herzschlag erneut überwunden werden müssen.

Des Weiteren hat es sich gezeigt, dass durch den Einsatz zumindest einer Trennwand in dem distalen. Bereich des Transplantates auch größere Konuswinkel im Diffusorbereich verwendet werden können. Optimal scheint jedoch bei einem arteriovenösen Shunt ein Diffusor mit einem Konuswinkel von etwa 7° mit einer zusätzliche längs verlaufenden Trennwand zu sein, wodurch eine hämodynamisch optimale Variante erreicht wird.

Für periphere vaskuläre Transplantate kann es vorteilhaft sein, dass sich an den Diffusorbereich ein distaler Endbereich bzw. ein Endrohr mit im Wesentlichen gleichem Durchmesser anschließt, der dem Durchmesser am Ende des Diffusorbereiches entspricht. Vorzugsweise ragt die Trennwand zumindest teilweise in diesen Endbereich hinein.

Die Erfindung ist in Ausführungsbeispielen anhand der Zeichnung näher erläutert. In dieser stellen dar:
Figur 1 ein vaskuläres röhrenförmiges Transplantat als arteriovenöser Shunt gemäß dem Stand der Technik;
Figur 2 einen schematischen Querschnitt durch einen Teil eines arteriovenösen Shunts mit einem distalen Diffusorbereich;
Figur 3 eine schematische Darstellung eines weiteren Ausführungsbeispiels eines arteriovenösen Shunts gemäß der Erfindung mit einem Diffusorbereich und einer in diesem Bereich vorgesehenen Trennwand;
Figur 4 ein Strömungsdiagramm im Bereich des Diffusors für eine arteriovenösen Shunt gemäß Figur 2;
Figur 5 ein Strömungsdiagramm im Bereich des Diffusors für einen arteriovenösen Shunt gemäß Figur 3;
Figur 6 eine teilweise geschnittene schematische Darstellang eines arteriovenösen Shunts in einer Ausführung gemäß Figur 3;
Figur 7 eine schematische teilweise geschnittene Darstellung eines peripheren vaskulären Transplantates gemäß der Erfindung; und
Figur 8 eine schematische Darstellung eines weiteren Ausführungsbeispiels eines arteriovenösen Shunts gemäß der Erfindung mit einem Diffusorbereich und zwei in diesem Bereich vorgesehenen Teiltrennwänden.

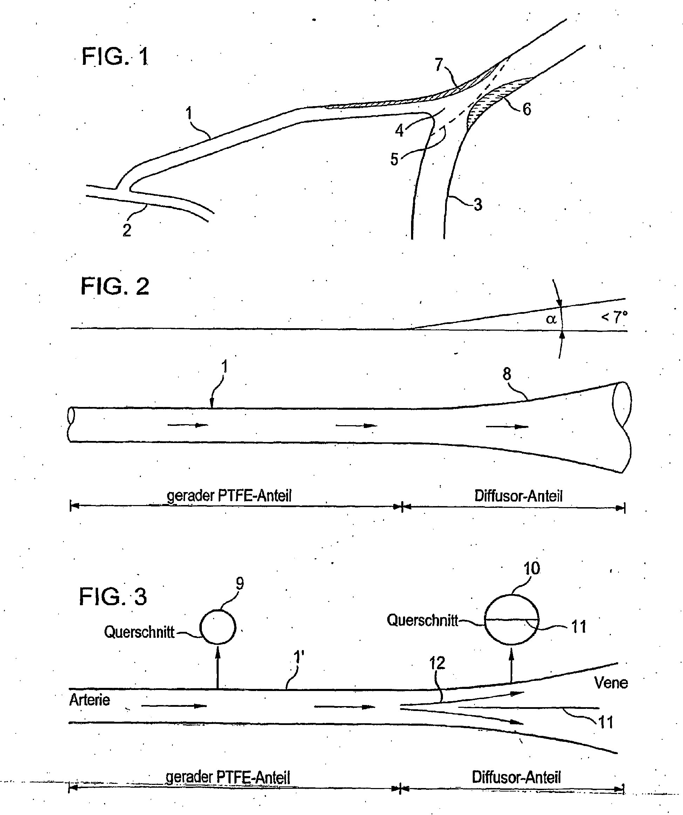

In Figur 1 ist ein arteriovenöser Shunt 1 gezeigt, der eine Arterie 2 mit einer Vene 3 verbindet. Der Shunt 1 ist ein vaskuläres röhrenförmiges Transplantat aus ePTFE, das an seinem proximalen Ende von der Arterie 2 abzweigt und im Wesentlichen mit gleichem Durchmesser in die Vene 3 führt, wobei die Verbindung zwischen dem Shunt 1 und der Vene über einen Flansch 4 erfolgt, der z. B. durch eine gestrichelt dargestellte Naht 5 mit der Vene 3 verbunden ist. Wie oben äusgeführt, bildet sich durch den schräg einfallenden Zentralstrom des Blutsam Venenboden allmählich eine Intimahyperplasie 6 aus, die durch die hohen Wandscherstresse ausgelöst wird. Parallel hierzu bildet sich in dem Shunt 1 im distalen Bereich eine Pseudointimahyperplasie 7, die im Wesentlichen durch Separationszonen in der Blutströmung ausgelöst wird.

In Figur 2 ist schematisch ein Teil eines arteriovenösen Shunts 1 dargestellt. Ausgehend von der hier nicht gezeigten Arterie ist dieser Shunt 1 eine Röhre mit im Wesentlichen konstantem Durchmesser, der sich im distalen, der Vene zugewandten Bereich nach Art. sors 8 mit einem Konuswinkel α von etwa 6° bis 7° erweitert. Nicht gezeigt in dieser Darstellung ist gegebenenfalls ein Flansch, um den Shunt 1 mit der Vene nach Art der Figur 1 zu verbinden.

In Figur 3 ist eine zweite Ausführungsform eines arteriovenösen Shunts 1' dargestellt, der, von der Arterie ausgehend, zunächst einen im Wesentlichen konstanten Querschnitt 9 aufweist und dann, wie bei der Ausführung gemäß Figur 2, in einen Diffusor 8 mit ebenfalls geringem Konuswinkel von 6° bis 7° übergeht. Der sich stetig vergrößernde Querschnitt des Diffusors 8 ist mit 10 bezeichnet. In diesem Diffusor 8 ist eine ebene Trennwand 11 angeordnet, die in diesem Falle etwa in der Mitte des Diffusors 8 verläuft und mit gegenüber liegenden Wandbereichen des Diffusors 8 verbunden ist, wie auch aus dem Querschnitt 10 hervorgeht. Wie durch Pfeile 12 angedeutet, wird die Blutströmung durch die Trennwand 11 aufgespalten. Die Blutströmung ist laminar, sodass keine Wirbel oder Separationszonen entstehen. Durch die Aufweitung des Diffusors 8 wird außerdem die Strömungsgeschwindigkeit verlangsamt, sodass die Wandscherstresse am Boden der Vene 3 deutlich geringer werden und sich keine Intimahyperplasie auf dem Boden der Vene 3 und keine Pseudointima in dem Shunt aufbauen.

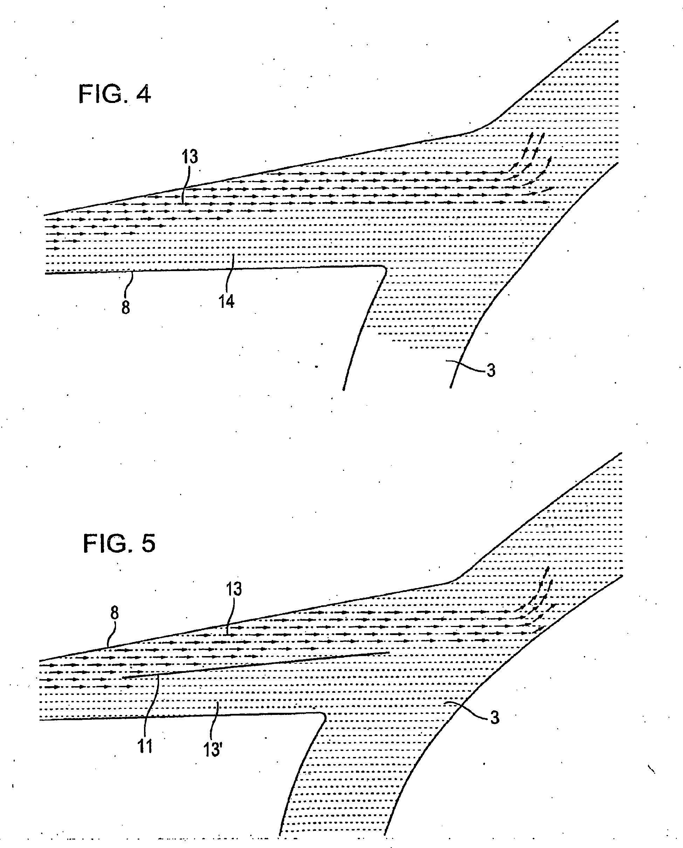

In Figur 4 ist ein Strömungsdiagramm (Particle Image Velocimetry - Teilchen Bild-Geschwindigkeitsmessung) für einen Shunt gemäß Figur 2 dargestellt. Gezeigt sind lediglich der Diffusor 8 des Shunts und ein Teil der Vene 3. In Figur 4 sind laminare Strömungsbereiche durch geschwärzte kurze Linien dargestellt, während langsamere Strömungen durch Punkte angedeutet sind. Man sieht, dass sich in der in Figur 4 oberen Hälfte eine stabile laminare Strömung 13 ausbildet, wohingegen im gegenüber liegenden, in der Figur 4 unteren Bereich noch eine gewisse Separationszone 14 vorhanden ist. Gleichwohl wird durch die Verlangsamung der Zentralströmung des Blutes der Scherstress am Boden der Vene 3 verringert, sodass die Ausbildung der Intimahyperplasie an diesem Ort ebenfalls deutlich verringert wird. Durch die laminare Strömung wird auch die Ausbildung der Pseudointima im Inneren des Shunts 1 deutlich verringert.

In Figur 5 ist das Strömungsdiagramm für einen Shunt 1' gemäß der Figur 3 mit einer ebenen Trennwand 11 gezeigt. Hier ist deutlich sichtbar, dass die Zentralströmung zu beiden Seiten der Trennwand 11 in laminare Strömungen 13 beziehungsweise 13' übergeht, sodass auch im Bereich der Strömung 13' praktisch keine Separationszonen auftreten. Ebenso wird der Wandscherstress auf den Boden der Vene 3 verringert. Eine Intimahyperplasie und eine Pseudointima werden im Wesentlichen nicht oder nur in geringem Maße aufgebaut.

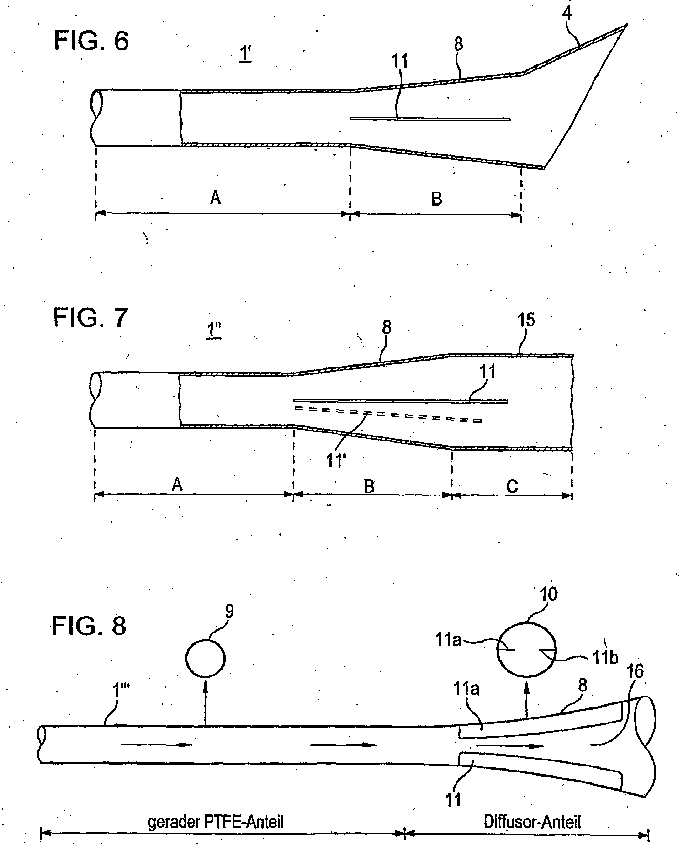

In Figur 6 ist eine teilweise geschnittene Ansicht des Shunts 1' gemäß Figur 3 mit einer - ebenen Trennwand 11 im Bereich des Diffusors 8 dargestellt, wobei hier ein Flansch 4 zur Verbindung mit der Vene nur angedeutet ist. In diesem Falle ist der Konuswinkeln des Diffusors ebenfalls 6° bis 7°, wobei aufgrund der Trennwand 11 auch höhere Werte möglich sind. Angedeutet sind noch der "gerade" Bereich A des Shunts und der sich konisch erweiternde Diffusorbereich B.

In Figur 7 ist schematisch ein vaskuläres Transplantat 1" dargestellt, das insbesondere für periphere Transplantate geeignet ist. Dieses Transplantat 1" weist zunächst, ausgehend von seinem proximalen Ende, einen "geraden" Bereich A, anschließend einen Diffusorbereich B mit dem sich konisch erweiternden Diffusor 8 und schließlich im distalen Bereich einen sich an den Diffusors 8 anschließenden weiteren "geraden" Bereich C auf. Der Bereich C wird durch ein Endrohr 15 gebildet, das einen Durchmesser aufweist, der dem Durchmesser am Ende des Diffusors 8 entspricht.

Auch für dieses Transplantat 1" ist eine Trennwand 11 vorgesehen, die sich. über den Diffusorbereich B und zumindest einen Teil des zweiten "geraden" Bereichs C in das Endrohr 15 erstreckt. Nicht dargestellt ist für dieses Transplantat ein sich an das Endrohr 15 anschließender Flansch zum Verbinden mit einem empfangenden Gefäß zum Beispiel durch eine Naht etc.

Angedeutet ist für dieses Transplantat 1" noch eine zweite ebene Trennwand 11', die gegenüber liegende Wandbereiche des Shunts im Diffusorbereich B und im zweiten "geraden" Bereich C miteinander verbindet. Diese zusätzliche Trennwand 11' kann gegenüber der Trennwand 11 geneigt angeordnet sein, wobei hier jedoch andere Ausgestaltungen entsprechend den Anforderungen hinsichtlich der Verlangsamung und Laminarisierung der Strömung möglich sind.

In Figur 8 ist eine schematische Darstellung eines weiteren Ausführungsbeispiels eines arteriovenösen Shunts 1"' ähnlich wie in Figur 3 dargestellt. Dieser Shunt 1''' weist, von der Arterie ausgehend, zunächst wiederum einen im Wesentlichen konstanten Querschnitt 9 auf, der sich dann im Bereich des Diffusors 8 kontinuierlich konisch vergrößert und in Figur 8 mit 10 bezeichnet ist. In dem Diffusor 8 sind ausgehend von zwei gegenüber liegenden Wandbereichen zwei segelartige Trennwände 11a und 11b angeordnet, die in den Diffusor hineinragen, wobei zwischen den inneren Rändern der Teil-Trennwände 11a und 11b ein sich konisch erweiternder Spalt 16 verbleibt. Auch mit einer solchen Konfiguration können Separationszonen in dem Transplantat vermieden werden; ebenso werden keine Wirbel erzeugt. Hiermit werden die Wandscherstresse am Boden der Vene deutlich geringer; es baut sich keine oder nur eine geringe Intimahyperplasie auf dem Boden der Vene und keine Pseudointima in dem Shunt auf. Ebenso bleibt der Diffusoreffekt im Wesentlichen erhalten.

Auch wenn im Vorhergehenden im Wesentlichen ebene Trennwände beschrieben worden sind, sind auch andere Ausführungen möglich: So können zum Beispiel die Oberflächen der Trennwände strukturiert sein, zum Beispiel mit Mikrostrukturen in Form von Mikronoppen versehen sein, um eine glatte Strömung ohne Anhaften an den Trennwänden zu erreichen. Solche Mikrostrukturen, zum Beispiel nach Art des Lotuseffektes oder einer Haifischhaut, sind in anderen Bereichen der Technik bereits bekannt.

## Patentansprüche

1. Vaskulares röhrenförmiges Transplantat zum Verbinden von Gefäßen im menschlichen Körper für einen arteriovenösen Shunt zum Durchführen einer Dialyse, wobei das Transplantat mit seinem ersten proximalen Ende mit einem Gefäß und mit seinem distalen Ende an einem anderen Ort desselben Gefäßes oder mit einem anderen Gefäß verbunden ist, **dadurch gekennzeichnet, dass** das Lumen des Transplantats (1, 1', 1", 1"') im Bereich seines distalen Endes sich stetig konisch erweitert und dort einen Diffusor (8) bildet, wobei in dem Bereich des Diffusors (8, B) des Transplantats (1', 1", 1"') zumindest eine in der zentralen Strömungsrichtung des Blutes verlaufende Trennwand (11, 11', 11a, 11b) vorgesehen ist.

2. Transplantat nach Anspruch 1, **dadurch gekennzeichnet, dass** die zumindest eine Trennwand (11, 11', 11a, 11b) eben ist.

3. Transplantat nach Anspruch 1 oder 2, **dadurch gekennzeichnet, dass** die zumindest eine Trennwand (11, 11') gegenüber liegende Wandbereiche des Diffusors (8) miteinander verbindet.

4. Transplantat nach einem der vorhergehenden Ansprüche, **dadurch gekennzeichnet, dass** die Trennwand (11a, 11b), ausgehend von zumindest einem Wandbereich des Diffusors (8) sich segelartig in den Diffusor (8) erstreckt.

5. Transplantat nach Anspruch 4, dadurch gekenntzeichnet, dass die Trennwand (11a, 11b) aus zwei jeweils von gegenüber liegenden äußeren Wandbereiches des Diffusors (9) ausgehenden segelartigen, in den Diffusor (8) hineinragenden Teil-Trennwänden (11a, 11b) besteht, zwischen deren inneren Rändern ein Zwischenraum (16) verbleibt.

6. Transplantat nach einem der vorhergehenden Ansprüche, **dadurch gekennzeichnet, dass** die zumindest eine Trennwand (11, 11', 11a, 11b) in dem Bereich des Diffusors (8) in einer Ebene liegt, die die zentrale Strömungsrichtung des Blutes enthält oder parallel beziehungsweise geneigt zu dieser angeordnet ist.

7. Transplantat nach einem der vorhergehenden Ansprilche, **dadurch gekennzeichnet, dass** sich an den Diffusor (8) ein distaler Endbereich (C, Endrohr 15) mit im Wesentlichen konstantem Durchmesser anschließt, der dem Durchmesser am Ende des Diffusors (8) entspricht, und dass die zumindest eine Trennwand (11, 11') zumindest teilweise in diesen Endbereich (C, 15) hineinragt.

8. Transplantat nach einem der vorhergehenden Ansprüche, **dadurch gekennzeichnet, dass** der Konuswinkel (α) der Wand des Diffusors (8) in Bezug zu der zentralen Strömungsrichtung des Blutes in dem Transplantat (1', 1", 1"') so gewählt ist, dass es zu keinen oder nur geringen Totwasser- oder Separationszonen kommt, in denen das Blut nur sehr langsam fließt.

9. Transplantat nach Anspruch 8, **dadurch gekennzeichnet, dass** der Konuswinkel (α) des Diffusors (8) in Bezug zu der zentralen Strömungsrichtung des Blutes in dem Transplantat (1', 1", 1"') zwischen 5° und 15° liegt.

10. Transplantat nach Anspruch 8 oder 9, **dadurch gekennzeichnet, dass** der Konuswinkel (α) des Diffusors (8) kleiner als 10°, vorzugsweise 6° bis 7° ist.

11. Transplantat nach einem der vorhergehenden Ansprüche, **dadurch gekennzeichnet, dass** das Transplantat aus expandiertem Polytetrafluorethylen (ePTFE-Transplantat) besteht.

## Claims

1. A vascular tubular transplant for connecting vessels in the human body for an arteriovenous shunt for carrying out a dialysis, the transplant being connected to a vessel with its first proximal end and to another location of the same vessel or to another vessel with its distal end, **characterized in that** the lumen of the transplant (1, 1', 1'', 1"') widens in a steady conical manner in the region of its distal end and forms a diffuser (8) there, at least one dividing wall (11, 11', 11a, 11b) extending in the central direction of flow of the blood being provided in the region of the diffuser (8, B) of the transplant (1, 1', 1", 1"').

2. The transplant according to claim 1, **characterized in that** the at least one dividing wall (11, 11', 11a, 11b) is plane.

3. The transplant according to claim 1 or 2, **characterized in that** the at least one dividing wall (11, 11') connects opposite wall areas of the diffuser (8) with each other.

4. The transplant according to any of the preceding claims, **characterized in that** the dividing wall (11a, 11b extends in s sail-like fashion into the diffuser (8) starting from at least one wall area of the diffuser (8).

5. The transplant according to claim 4, **characterized in that** the dividing wall (11a, 11b) consists of two opposite sail-like partial dividing walls (11a, 11b) projecting into the diffuser (8) and starting in each case from opposite outer wall areas of the diffuser (8), a space (16) remaining between the inner edges of which.

6. The transplant according to any of the preceding claims, **characterized in that** the at least one dividing wall (11, 11', 11a, 11b) is in one plane in the area of the diffuser (8) which includes the central direction of flow of the blood or is disposed in parallel and/or inclined with respect to the same.

7. The transplant according to any of the preceding claims, **characterized in that** a distal end (C, end tube 15) having a substantially constant diameter corresponding to the diameter at the end of the diffuser (8) adjoins the diffuser (8) and that the at least one dividing wall (11, 11') projects into this end region (C, 15) at least partially.

8. The transplant according to any of the preceding claims, **characterized in that** the cone angle (α) of the wall of the diffuser (8) is chosen with respect to the central direction of flow of the blood in the transplant (1, 1', 1", 1"') such that dead fluid or separation zones in which the blood only flows very slowly do not or only insignificantly occur.

9. The transplant according to claim 8, **characterized in that** the cone angle (α) of the diffuser (8) is between 5° and 15° with respect to the central direction of flow of the blood in the transplant (1, 1', 1", 1"').

10. The transplant according to claim 8 or 9, **characterized in that** the cone angle (α) of the diffuser (8) is smaller than 10°, preferably 6° to 7°.

11. The transplant according to any of the preceding claims, **characterized in that** the transplant consists of expanded polytetrafluoroethylene (ePTFE transplant).

## Revendications

1. Greffon tubulaire vasculaire pour relier des vaisseaux dans le corps humain pour un accès artério-veineux pour effectuer une dialyse, le greffon étant relié par sa première extrémité proximale à un vaisseau et par son extrémité distale à un autre emplacement du même vaisseau ou à un autre vaisseau, **caractérisé en ce que** le lumen du greffon (1, 1', 1", 1"') s'évase en forme de cône de manière continue dans la zone de son extrémité distale et forme à cet endroit un diffuseur (8), au moins une paroi de séparation (11, 11', 11a, 11b), qui s'étend dans la direction d'écoulement centrale du sang, étant prévue dans la zone du diffuseur (8, B) du greffon (1, 1', 1 ", 1 "').

2. Greffon selon la revendication 1, **caractérisé en ce que** ladite au moins une paroi de séparation (11, 11', 11 a, 11b) est plane.

3. Greffon selon la revendication 1 ou 2, **caractérisé en ce que** ladite au moins une paroi de séparation (11, 11',) relie l'une à l'autre des zones de paroi opposées du diffuseur (8).

4. Greffon selon l'une des revendications précédentes, **caractérisé en ce que** partant d'au moins une zone de paroi du diffuseur (8), la paroi de séparation (11 a, 11 b) s'étend à la manière d'une voile dans le diffuseur (8).

5. Greffon selon la revendication 4, **caractérisé en ce que** la paroi de séparation (11a, 11b) est constituée par deux parois de séparation partielles (11a, 11b) qui partent chacune, à la manière d'une voile, de zones de paroi extérieures opposées du diffuseur (8) et qui font saillie dans le diffuseur (8), entre les bords intérieurs desquelles demeure un espace intermédiaire (16).

6. Greffon selon l'une des revendications précédentes, **caractérisé en ce que** ladite au moins une paroi de séparation (11, 11', 11a, 11b) se trouve, dans la zone du diffuseur (8), dans un plan qui contient la direction d'écoulement centrale du sang ou qui est agencé parallèlement ou en inclinaison par rapport à celle-ci, respectivement.

7. Greffon selon l'une des revendications précédentes, **caractérisé en ce qu'**au diffuseur (8) se raccorde une zone d'extrémité distale (C, tube d'extrémité 15) avec un diamètre sensiblement constant qui correspond au diamètre à l'extrémité du diffuseur (8), et **en ce que** ladite au moins une paroi de séparation (11, 11') fait saillie au moins en partie dans cette zone d'extrémité (C, 15).

8. Greffon selon l'une des revendications précédentes, **caractérisé en ce que** l'angle de conicité (α) de la paroi du diffuseur (8) par rapport à la direction d'écoulement centrale du sang dans le greffon (1'. 1", 1"') est choisi tel qu'il ne se produit aucune ou que de faibles zones de remous ou de séparation dans lesquelles le sang ne s'écoule que très lentement.

9. Greffon selon la revendication 8, **caractérisé en ce que** l'angle de conicité (α) du diffuseur (8) par rapport à la direction d'écoulement centrale du sang dans le greffon (1'. 1", 1 "') est située entre 5° et 15°.

10. Greffon selon la revendication 8 ou 9, **caractérisé en ce que** l'angle de conicité (α) du diffuseur (8) est inférieur à 10°, de préférence de 6° à 7°.

11. Greffon selon l'une des revendications précédentes, **caractérisé en ce que** le greffon est en polytétrafluoroéthylène expansé (greffon ePTFE).
